# EUROPEAN PATENT APPLICATION

(11) **EP 3 988 636 A1**
(43) Date of publication of application: **27.04.2022**
(21) Application number: 20825878.0
(22) Date of filing: 15.06.2020
(51) Int. Cl.: C12M 1/00

(54) **CELL CULTURE DEVICE**

(30) Priority: 20.06.2019 JP 2019114220
(71) Applicant: Sinfonia Technology Co., Ltd., Tokyo 105-8564 (JP)
(72) Inventor: SHINOHARA, Kenji, Tokyo 105-8564 (JP); YAMADA, Takayuki, Tokyo 105-8564 (JP); NISHIKAWA Yoshihiko, Tokyo 105-8564 (JP)
(74) Representative: Angerhausen, Christoph
(86) International application number: PCT/JP2020/023461
(87) International publication number: WO 2020/255929

(57) **Abstract**

A cell culture device for culturing cells in a closed container kept in an aseptic state includes: a chamber including an opening/closing part capable of being opened and closed; a connection path configured to connect closed containers accommodated in the chamber to each other and each having an inside kept in an aseptic state; a driving part configured to move cells from one closed container to the other closed container via the connection path; and a container attachment/detachment device. After the cells are moved from one closed container to the other closed container, the container attachment/detachment device is configured to remove one closed container from the connection path while maintaining an aseptic state inside the other closed container and the connection path, and connect a new closed container loaded into the chamber to the connection path while maintaining an aseptic state inside the new closed container.

## Description

### TECHNICAL FIELD

The present disclosure relates to a cell culture device for culturing cells.

### BACKGROUND

Generally, during cell culture, medium replacement for discharging an old medium and injecting a new medium is performed periodically. In addition, when cells proliferate along with the progress of culture, subculture is performed in which the proliferated cells are transferred to a new medium at a predetermined density and subjected to culture again.

In such cell culture, it is required to maintain an aseptic state. In particular, when an operator performs medium replacement or subculture, the risk of contamination of a culture system with various germs increases. Therefore, conventionally, a closed cell culture device capable of maintaining an aseptic state has been developed. For example, Patent Document 1 discloses a cell culture device capable of automatically performing medium replacement and subculture under aseptic conditions without the intervention of an operator.

### [Prior Art Document]

### Patent Document

Patent Document 1: Japanese laid-open publication No. 2017-6058

In the configuration of Patent Document 1, a culture container for initially culturing cells and a culture container to which cells are transferred for subculture are installed in advance inside the device, and the respective culture containers are connected by a tube or the like, thereby creating a closed system that maintains an aseptic state. The entire process of cell culture, including subculture, is performed in the closed system kept in an aseptic state. Therefore, all the culture containers cannot be carried into and out of the device until the entire process is completed. That is, the number of subculture containers installed in the device in advance is the limit number of subcultures.

In this regard, the number of subcultures in the cell culture process varies depending on the culture conditions such as the cell type, the container size, the culture environment, the culture purpose, and the like. In order to cope with cell culture under various conditions, an automatic culture method assuming multiple subcultures is required. In the device of Patent Document 1, in order to meet such needs, a number of culture containers just as much as the number of subcultures have to be installed inside the device in advance. However, in this case, a space for installing the culture containers for subcultures needs to be secured in the device, and the size of the device becomes large.

The present disclosure provides some embodiments of a closed cell culture device that eliminates the need to limit the number of subcultures while suppressing an increase in the size of the device.

### SUMMARY

According to one embodiment of the present disclosure, there is provided a cell culture device for culturing cells in a closed container whose inside is kept in an aseptic state, including: a chamber including an opening/closing part capable of being opened and closed; a connection path configured to connect at least two closed containers accommodated in the chamber to each other and each having an inside kept in an aseptic state; a driving part configured to move cells between the at least two closed containers via the connection path; and a container attachment/detachment device configured to remove one of the at least two closed containers as a cell movement source from the connection path while maintaining an aseptic state inside another of the at least two closed containers to which the cells are moved by the driving part and inside the connection path, and connect a new closed container loaded into the chamber via the opening/closing part to the connection path instead of the removed closed container while maintaining an aseptic state inside the new closed container and the connection path.

According to such a configuration, when the cells have been cultured in any of the closed containers, the driving part can be driven to move the cells from the closed container to another closed container via the connection path, whereby the subculture can be performed. When the subculture is completed, the empty closed container can be unloaded from the chamber to the outside of the chamber through the opening/closing part, and a new closed container can be loaded into the chamber through the opening/closing part. The new closed container can be connected to the connection path by the container attachment/detachment device while maintaining an aseptic state. Therefore, even when the subculture is performed a plurality of times, it is not necessary to prepare closed containers to be used for the second and subsequent subcultures in the chamber in advance, which makes it possible to suppress an increase in the size of the cell culture device. Further, by repeating the replacement of the closed container, it is possible to cope with the increase in the number of subcultures, whereby the limit on the number of subcultures is substantially eliminated.

Further, in the cell culture device, the chamber may include a first chamber having a first opening/closing part and configured to accommodate at least one closed container therein and a second chamber having a second opening/closing part and configured to accommodate at least one closed container therein, and an environment adjustment part configured to adjust internal environments of the first chamber and the second chamber may be provided.

In general, cells are affected by a culture environment. Therefore, in order to properly culture the cells, it is required to keep the internal environment of the cell culture device constant. In the cell culture device according to the present disclosure, the opening/closing part of the chamber is opened at the time of replacing the closed container. Therefore, it is difficult to keep the internal environment of the chamber of the cell culture device constant.

According to the above-described configuration, for example, when subculture is performed from the closed container accommodated in the first chamber to the closed container accommodated in the second chamber, the closed container can be replaced by opening only the first opening/closing part of the first chamber. At this time, if the second opening/closing part of the second chamber is kept in a closed state, the internal environment of the second chamber in which the closed container for performing a cell culture after the subculture is installed can be kept constant without being affected by the outside of the cell culture device.

Further, the cell culture device may further include: temperature sensors configured to detect temperatures inside the first chamber and the second chamber, wherein the environment adjustment part is configured to adjust the temperatures inside the first chamber and the second chamber, and the environment adjustment part adjusts the temperatures inside the first chamber and the second chamber to predetermined values when the cells are moved through the connection path.

According to the above-described configuration, the cells are moved between the closed containers installed in the respective chambers when the temperatures inside the first chamber and the second chamber are equal to predetermined temperatures, whereby the cell culture temperature can be kept constant before and after subculture.

According to the present disclosure, it is possible to provide a closed cell culture device that eliminates the need to limit the number of subcultures while suppressing an increase in the size of the device.

### BRIEF DESCRIPTION OF DRAWINGS

FIG. 1 is a schematic front view showing the configuration of a cell culture device according to an embodiment of the present disclosure.
FIG. 2 is a front view showing the configuration of a culture part.
FIG. 3 is a diagram showing a culture circuit formed inside the cell culture device.
FIG. 4 is a flowchart showing the respective steps of a cell culture process performed by the cell culture device.

### DETAILED DESCRIPTION

Embodiments of the present disclosure will now be in detail described in detail with reference to the accompanying drawings.

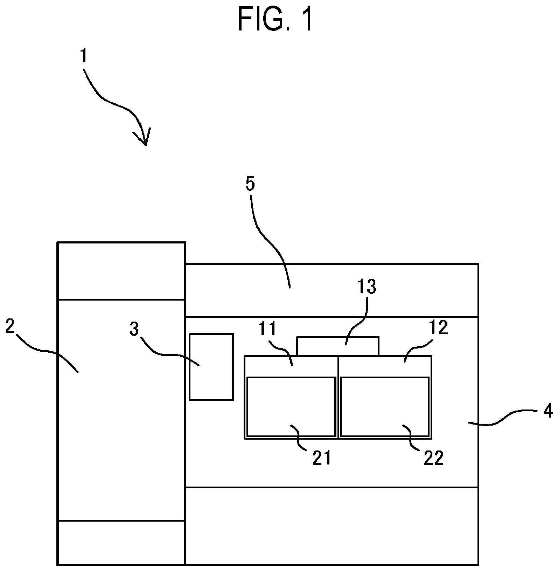

As shown in FIG. 1, the cell culture device 1 according to the present embodiment includes a refrigerating storage part 2, a heating storage part 3, a culture part 4, and a controller 5. The cell culture device 1 is a device for culturing cells according to the data inputted to and stored in the controller 5. In the following description, the front-rear direction is defined as a direction perpendicular to the drawing sheet surface in FIG. 1.

The refrigerating storage part 2 and the heating storage part 3 are housings in which shelves for arranging containers containing media or reagents are formed. Although not shown, the front surfaces of the refrigerating storage part 2 and the heating storage part 3 are provided with doors that can open and close the openings formed on the front surfaces of the housings. The refrigerating storage part 2 is provided with a cooling mechanism (not shown), and the internal temperature thereof is maintained at an arbitrary temperature lower than the room temperature. The heating storage part 3 is arranged inside the culture part 4, and the temperature inside the heating storage part 3 is substantially equal to the temperature inside the culture part 4. Further, tubes are connected to the container arranged inside the refrigerating storage part 2 and the container arranged inside the heating storage part 3 so that the liquids inside the containers can flow out through the tubes. The liquids inside the containers can be discharged by a pump 102 described later. The container arranged inside the refrigerating storage part 2 is a large container such as a tank or the like. Examples of the container arranged inside the heating storage part 3 include a bottle, a bag and the like.

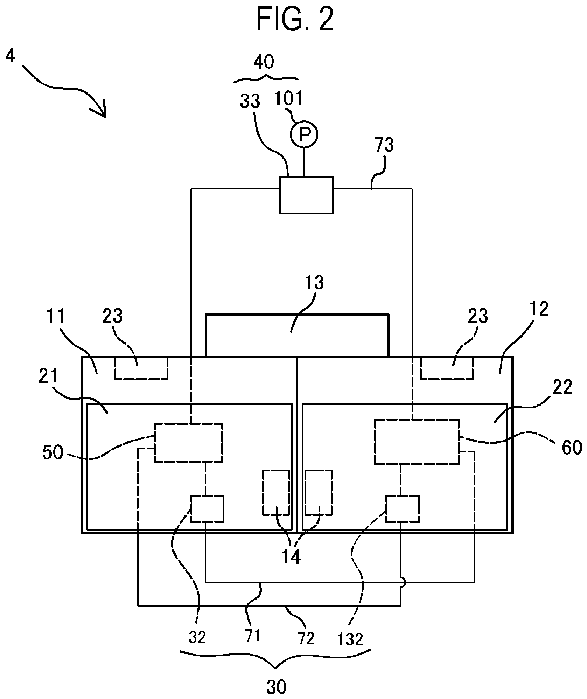

As shown in FIGS. 1 and 2, the culture part 4 includes a first chamber 11 having a first opening/closing part 21 on the front surface thereof, a second chamber 12 having a second opening/closing part 22 on the front surface thereof, an environment adjustment part 13, and container attachment/detachment devices 14. The wall surfaces of the first chamber 11 and the second chamber 12, and the first opening/closing part 21 and the second opening/closing part 22 are made of a heat insulating material. As a result, the temperatures inside the first chamber 11 and the second chamber 12 are kept constant in a state in which the first opening/closing part 21 and the second opening/closing part 22 are closed. Further, as shown in FIG. 2, a closed container 50 is installed inside the first chamber 11, and a closed container 60 is installed inside the second chamber 12. The inside of the closed containers 50 and 60 is aseptic. Examples of the container include a flask, a multi-layer container, a bag, and the like. Further, the volume of the closed container 60 is larger than the volume of the closed container 50. This is because when the cells cultured in the closed container 50 and having a high concentration are transferred to the closed container 60 and further cultured in the closed container 60, the amount of the medium contained in the closed container 60 needs to be larger than the amount of the medium contained in the closed container 50. The dotted line portions in FIG. 2 mean that certain components are arranged inside the first chamber 11 and the second chamber 12.

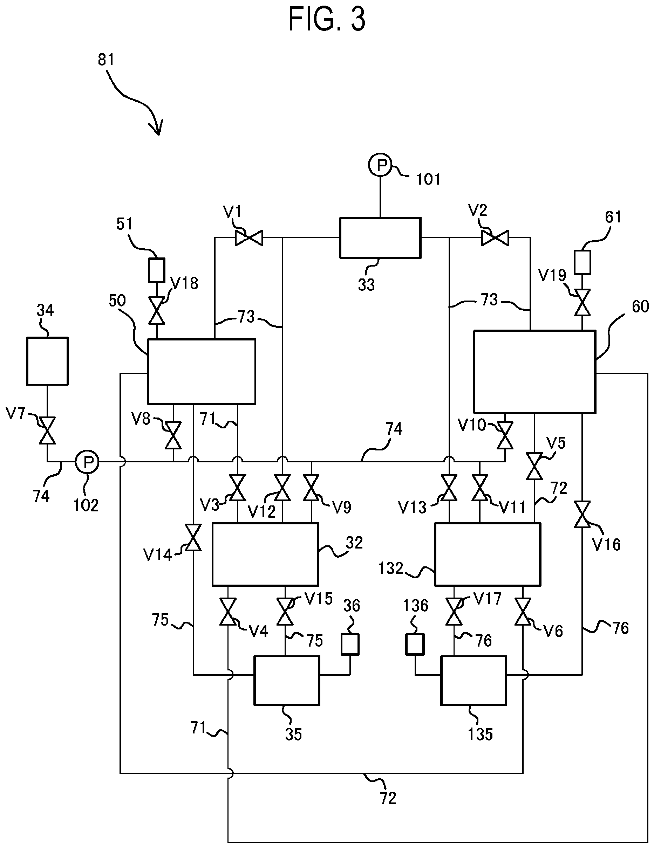

Further, as shown in FIG. 3, a degassing part 51 communicating with the outside air is attached to the closed container 50, and a degassing part 61 communicating with the outside air is attached to the closed container 60. A valve V18 is arranged between the closed container 50 and the degassing part 51. When the valve V18 is in an open state, the gas inside the closed container 50 is released to the atmosphere through the degassing part 51. Further, a valve V19 is arranged between the closed container 60 and the degassing part 61. When the valve V19 is in an open state, the gas inside the closed container 60 is released to the atmosphere through the degassing part 61. As the valve V18 and the valve V19, for example, pinch valves may be adopted.

The environment adjustment part 13 includes a built-in heating device and a CO₂ supply device and can adjust the temperatures and CO₂ concentrations inside the first chamber 11 and the second chamber 12 according to a signal sent from the controller 5. Further, sensors 23 for detecting the temperatures and CO₂ concentrations are arranged inside the first chamber 11 and the second chamber 12. The information detected by the sensors 23 is outputted to the controller 5. Devices for adjusting other internal environments may be built in the environment adjustment part 13. In this case, the sensors 23 are sensors that can also detect other internal environments.

Further, as shown in FIG. 2, the culture part 4 includes a connection path 30 for connecting the closed container 50 and the closed container 60 to each other, and a driving part 40 for moving cells between the closed container 50 and the closed container 60 via the connection path 30. The connection path 30 includes tubes 71 and 72 whose inside is aseptic and stirring parts 32 and 132 whose inside is aseptic. The stirring part 32 is arranged inside the first chamber 11 and is connected to the closed containers 50 and 60 via the tube 71. The stirring part 132 is arranged inside the second chamber 12 and is connected to the containers 50 and 60 via the tube 72. Further, the driving part 40 includes a pump 101 and a gas tank 33 connected to the pump 101 and containing a gas therein. The gas tank 33 is connected to the stirring parts 32 and 132 and the closed containers 50 and 60 via the tube 73. The gas contained in the gas tank 33 may be, for example, CO₂, and may be another gas or a mixed gas composed of a plurality of types of gases. In FIG. 2, the illustration of a tube 73 for connecting the gas tank 33 and the stirring parts 32 and 132 is omitted. For the details thereof, FIG. 3 may be referred to.

As shown in FIG. 3, the medium container 34 arranged inside the heating storage part 3 is connected to the stirring parts 32 and 132 and the closed containers 50 and 60 via a tube 74. Further, a pump 102 is installed between the medium container 34 and a group including the stirring parts 32 and 132 and the closed containers 50 and 60. By driving the pump 102, the medium contained in the medium container 34 can be supplied to the stirring parts 32 and 132 and the closed containers 50 and 60. The medium container 34 is connected to a medium tank (not shown) arranged inside the refrigerating storage part 2 via a tube. The medium stored in the refrigerating storage part 2 is appropriately supplied to the medium container 34 arranged in the heating storage part 3.

Further, a waste liquid container 35 is connected to the stirring part 32 and the closed container 50 via a tube 75. Further, a waste liquid container 135 is connected to the stirring part 132 and the closed container 60 via a tube 76. Moreover, by driving the pump 101, the liquid inside the stirring part 32 and the closed container 50 can be discharged to the waste liquid container 35, and the liquid inside the stirring part 132 and the closed container 60 can be discharged to the waste liquid container 135. A degassing part 36 communicating with the outside air is formed in the waste liquid container 35. The gas inside the waste liquid container 35 is released to the atmosphere through the degassing part 36. A degassing part 136 communicating with the outside air is formed in the waste liquid container 135. The gas inside the waste liquid container 135 is released to the atmosphere through the degassing part 136. Check valves (not shown) are provided between the waste liquid container 35 and the degassing part 36 and between the waste liquid container 135 and the degassing part 136, respectively, to prevent the outside air from entering the waste liquid containers 35 and 135.

In the following description, an internal space surrounded by the stirring parts 32 and 132, the gas tank 33, the medium container 34, the waste liquid containers 35 and 135, the closed containers 50 and 60, and the tubes 71 to 76 connecting them is referred to as a culture circuit 81. A liquid or a gas may flow through the culture circuit 81.

A valve V1 is installed in the tube 73 between the gas tank 33 and the closed container 50, and a valve V2 is installed in the tube 73 between the gas tank 33 and the closed container 60. Further, a valve V12 is installed in the tube 73 between the gas tank 33 and the stirring part 32, and a valve V13 is installed in the tube 73 between the gas tank 33 and the stirring part 132. A valve V3 is installed in the tube 71 between the stirring part 32 and the closed container 50, and a valve V4 is installed in the tube 71 between the stirring part 32 and the closed container 60. Further, a valve V5 is installed in the tube 72 between the stirring part 132 and the closed container 60, and a valve V6 is installed in the tube 72 between the stirring part 132 and the closed container 50. A valve V7 is installed in the tube 74 between the medium container 34 and the pump 102, a valve V8 is installed in the tube 74 between the closed container 50 and the pump 102, a valve V9 is installed in the tube 74 between the stirring part 32 and the pump 102, a valve V10 is installed in the tube 74 between the closed container 60 and the pump 102, and a valve V11 is installed in the tube 74 between the stirring part 132 and the pump 102. A valve V14 is installed in the tube 75 between the closed container 50 and the waste liquid container 35, and a valve V15 is installed in the tube 75 between the stirring part 32 and the waste liquid container 35. A valve V16 is installed in the tube 76 between the closed container 60 and the waste liquid container 135, and a valve V17 is installed in the tube 76 between the stirring part 132 and the waste liquid container 135. By opening and closing the valves V1 to V17, the flows of a liquid and a gas flowing through the tubes 71 to 76 can be permitted or cut off. As the valves V1 to V17, for example, pinch valves may be adopted.

As shown in FIG. 2, the container attachment/detachment devices 14 are arranged inside the first chamber 11 and the second chamber 12, respectively. The container attachment/detachment device 14 is a device that removes the closed container 50 or 60 from the culture circuit 81 while maintaining the aseptic state inside the culture circuit 81 and connects another new closed container to the culture circuit 81 while maintaining the aseptic state inside the culture circuit 81. Specifically, for example, when removing the closed container 50, the closed container 50 is removed from the tubes 72, 73 and 74, and the stirring part 32 is removed from the tubes 73 and 74 and the tube 71 having one end connected to the closed container 60. As a result, the closed container 50, the stirring part 32 and the waste liquid container 35 are integrally removed from the culture circuit 81. At the same time, a new closed container is attached to the tubes 72, 73 and 74, and a new stirring part is attached to the tubes 73 and 74 and the tube 71 having one end connected to the closed container 60. As a result, the new closed container, the new stirring part and the waste liquid container are integrally connected to the culture circuit 81. Examples of the container attachment/detachment device 14 include BioWelder (manufactured by Sartorius Stedim Japan) and OPTA aseptic connector (manufactured by Sartorius Stedim Japan). The container attachment/detachment device 14 may be arranged outside the culture part 4. In this case, the container attachment/detachment device 14 is loaded into the first chamber 11 or the second chamber 12 when the closed container is replaced. The container attachment/detachment device 14 may be loaded by an operator or may be loaded automatically. When the loading of the container attachment/detachment device 14 is performed automatically, for example, a robot arm or the like is separately arranged. In addition, when the container attachment/detachment device 14 is arranged outside the culture part 4, only one container attachment/detachment device 14 may be arranged.

The controller 5 controls the internal environments of the first chamber 11 and the second chamber 12 using the environment adjustment part 13, controls the driving of the pumps 101 and 102, controls the opening/closing of the valves V1 to V19, and controls the opening/closing of the first opening/closing part 21 and the second opening/closing part 22, based on the settings inputted in advance by the operator.

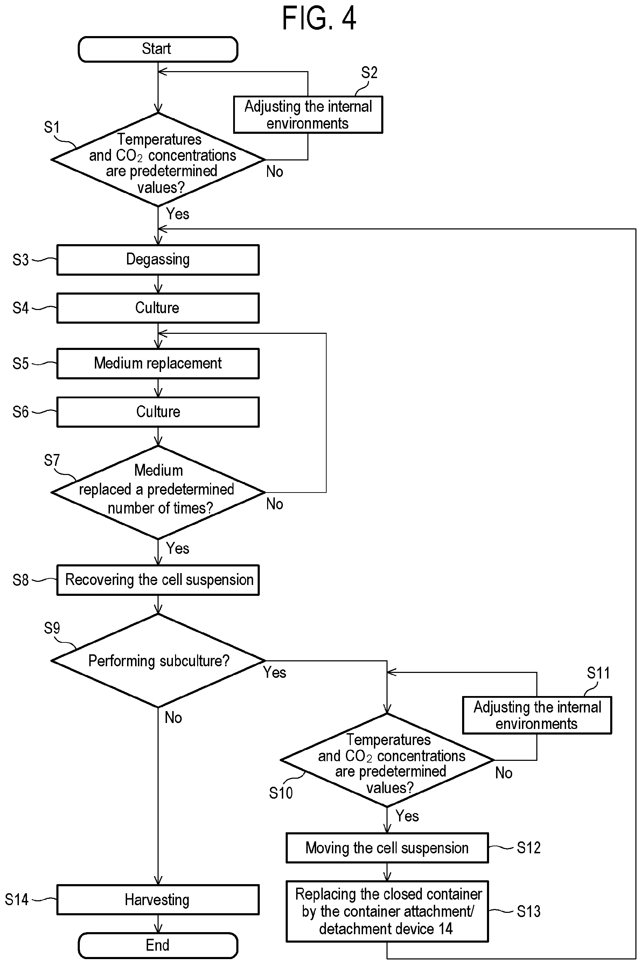

Hereinafter, a cell culture method using the cell culture device 1 according to the present embodiment will be described with reference to FIG. 4. The cell culture method using the cell culture device 1 includes a culture step, a medium replacement step, a cell suspension recovery step, and a subculture step. The culture step is a step of culturing cells in the closed container 50 or 60 at a predetermined temperature and a predetermined CO₂ concentration. The medium replacement step is a step of replacing the medium inside the closed container 50 or 60 in which the cells are cultured. The cell suspension recovery step is a step of recovering a cell suspension containing the cells and the medium from the inside of the closed container 50 or 60. The subculture step is a step of moving the cell suspension from the closed container 50 to the closed container 60.

The culture circuit 81 is set in advance in the cell culture device 1. Specifically, as shown in FIG. 3, the closed container 50 is installed inside the first chamber 11, the closed container 60 is installed inside the second chamber 12, and the medium container 34 is arranged inside the heating storage part 3. A cell suspension is accommodated inside the closed container 50. The medium container 34 arranged in the heating storage part 3 is connected to the medium tank arranged in the refrigerating storage part 2 via a tube (not shown). Further, the tubes 71 to 76 are set in the stirring parts 32 and 132, the gas tank 33, the medium container 34, the waste liquid containers 35 and 135, the closed containers 50 and 60, the valves V1 to V17 and the pumps 101 to 102 to form the culture circuit 81 (see FIG. 3). At this time, the inside of the culture circuit 81 is kept in an aseptic state, and all the valves are kept in a closed state.

Various settings such as the culture time in the culture step, the amount of the medium replaced in the medium replacement step and the amount of the medium supplied in the subculture step are inputted to the controller 5 in advance according to the experiments by the operator. The controller 5 automatically performs the respective steps based on the inputted settings.

When an input for starting culture is received in a state in which the culture circuit 81 is set and the first opening/closing part 21 of the first chamber 11 and the second opening/closing part 22 of the second chamber 12 are closed, the controller 5 of the cell culture device 1 performs a preliminary operation as a preceding step of the culture step. Specifically, as shown in FIG. 4, it is checked whether the temperatures and the CO₂ concentrations inside the first chamber 11 and the second chamber 12 detected by the sensors 23 are predetermined values (step S1). If the temperatures or the CO₂ concentrations inside the first chamber 11 and the second chamber 12 are not the predetermined values (S1: NO), the environment adjustment part 13 adjusts the temperatures and the CO₂ concentrations (step S2). The predetermined value of the temperature is, for example, 37 degrees C, and the predetermined value of the CO₂ concentration is, for example, 5%.

If the temperatures and the CO₂ concentrations inside the first chamber 11 and the second chamber 12 are predetermined values (S1: YES), the controller 5 opens the valve V18. As a result, when an excess gas (CO₂) remains inside the closed container 50, the excess gas is discharged from the degassing part 51 attached to the closed container 50 (step S3). Thereafter, the controller 5 closes the valve V18, thereby completing the preliminary operation.

A cell suspension may be contained in the closed container 50 between steps S1 and S3. In this case, for example, a cell suspension containing cells and a medium is contained in advance in a container which is arranged inside the heating storage part 3 and connected to the closed container 50 via a tube. Then, after step S1, the cell suspension contained in the container arranged inside the heating storage part 3 in advance is sent to the closed container 50 via the above-mentioned tube.

### (Culture Step)

The cell culture device 1 performs cell culture while monitoring the temperature and the CO₂ concentration inside the first chamber 11 by the sensor 23 and keeping the temperature and the CO₂ concentration inside the first chamber 11 at predetermined values by the environment adjustment part 13 (step S4). When a preset culture time elapses, the culture step comes to an end.

Before the end of the culture step, as a pre-stage of the medium replacement step, the controller 5 supplies a fresh medium required for medium replacement from the medium tank (not shown) arranged inside the refrigerating storage part 2 to the medium container 34 arranged inside the heating storage part 3 via a tube. The fresh medium supplied to the medium container 34 is heated in the heating storage part 3 until the temperature thereof becomes equal to the predetermined temperature inside the first chamber 11 where the culture is performed.

### (Medium Replacement Step)

After the culture step is completed, the medium replacement step (step S5) is performed. First, the controller 5 opens the valves V1 and V14 and drives the pump 101 to allow the CO₂ existing inside the gas tank 33 to flow into the closed container 50. As a result, the medium inside the closed container 50 is discharged to the waste liquid container 35 through the tube 75. At this time, the gas inside the waste liquid container 35 is released to the atmosphere through the degassing part 36. Thereafter, the controller 5 stops the pump 101 and closes the valves V1 and V14. Next, the controller 5 opens the valves V7 and V8 and drives the pump 102 to supply a fresh medium from the medium container 34 to the closed container 50 via the tube 74. After supplying the fresh medium, the controller 5 stops the pump 102 and closes the valves V7 and V8.

After supplying the fresh medium to the closed container 50, the controller 5 opens the valve V18. As a result, when a gas remains inside the closed container 50, the gas is discharged from the degassing part 51 installed in the closed container 50. Thereafter, the controller 5 closes the valve V18 and completes the medium replacement step (step S5). The amount of the medium discharged from the closed container 50 to the waste liquid container 35 via the tube 75 and the amount of the fresh medium supplied from the medium container 34 to the closed container 50 via the tube 74 are set in advance.

When the medium replacement step (step S5) is completed, the culture step (step S6) is performed in the same manner as in step S4. After the culture step (step S6) is completed, it is checked whether the medium replacement has been performed a predetermined number of times set in advance (step S7). If the medium replacement has not been performed a predetermined number of times (S7: NO), the process returns to step S5, and the medium is replaced in the same manner as described above. If the medium replacement has been performed a predetermined number of times (S7: YES), the cell suspension recovery step and the subculture step described below are performed.

### (Cell Suspension Recovery Step)

First, the cell suspension containing the cells cultured in the closed container 50 is recovered (step S8). When recovering the cell suspension, the controller 5 opens the valves V1 and V3 and drives the pump 101 to allow the CO₂ inside the gas tank 33 to flow into the closed container 50. As a result, the cell suspension inside the closed container 50 moves to the stirring part 32 through the tube 71. After the cell suspension inside the closed container 50 is moved to the stirring part 32, the controller 5 stops the pump 101 and closes the valves V1 and V3. A small amount of the cell suspension whose concentration has become uniform by being stirred by the stirring part 32 is carried to a cell counting part (not shown) where the concentration of the cell suspension is measured. Subsequently, the controller 5 performs the supply of the medium to the stirring part 32 and/or the discharge of the excess cell suspension from the stirring part 32 so that the concentration of the cell suspension contained inside the stirring part 32 becomes equal to a preset concentration. When performing the supply of the medium, the controller 5 opens the valves V7 and V9 and drives the pump 102. As a result, a predetermined amount of medium is supplied from the medium container 34 to the stirring part 32 via the tube 74. When performing the discharge of the excess cell suspension, the controller 5 opens the valves V12 and V15 and drives the pump 101 to allow the CO₂ inside the gas tank 33 to flow into the stirring part 32. As a result, a part of the cell suspension inside the stirring part 32 is discharged to the waste liquid container 35 via the tube 75. At this time, the gas inside the waste liquid container 35 is released to the atmosphere through the degassing part 36. As a result, the concentration of the cell suspension contained in the stirring part 32 becomes equal to the preset concentration. Thereafter, the controller 5 stops the pump 102 and closes the valves V7 and V9, and/or stops the pump 101 and closes the valves V12 and V15. This completes the cell suspension recovery step (step S8).

### (Subculture Step)

Next, the controller 5 checks whether to perform the subculture step (step S9). When performing the subculture step (S9: YES), first, the controller 5 checks by the sensors 23 whether both the temperatures and the CO₂ concentrations inside the first chamber 11 and the second chamber 12 are predetermined values (step S10). If the temperatures or the CO₂ concentrations inside the first chamber 11 and the second chamber 12 are not the predetermined values (S10: NO), the temperatures and the CO₂ concentrations are adjusted by the environment adjustment part 13 (step S11). Thereafter, step S10 is performed again.

If both the temperatures and the CO₂ concentrations inside the first chamber 11 and the second chamber 12 are the predetermined values (S10: YES), the cell suspension inside the stirring part 32 is moved to the closed container 60 (step S12). Specifically, the controller 5 opens the valves V12 and V4 and drives the pump 101 to allow the CO₂ inside the gas tank 33 to flow into the stirring part 32 via the closed container 50. As a result, the cell suspension inside the stirring part 32 is moved to the closed container 60 through the tube 71. After the cell suspension is moved to the closed container 60, the controller 5 stops the pump 101 and closes the valves V12 and V4. Subsequently, the controller 5 opens the valve V19. As a result, when a gas remains inside the closed container 60, the gas is discharged from the degassing part 61 attached to the closed container 60. Thereafter, the controller 5 closes the valve V19, whereby step S12 is completed.

After the cell suspension is moved to the closed container 60 (step S12), the container attachment/detachment device 14 replaces the closed container 50, from which the cell suspension is removed, with another closed container (step S13). Specifically, first, the controller 5 opens the first opening/closing part 21 of the first chamber 11. Subsequently, a new closed container having an aseptic internal state and including a new stirring part and a new waste liquid container is loaded into the first chamber 11 from the outside of the cell culture device 1. In advance, the new closed container and the new stirring part are connected to each other via a tube, the new closed container and the new waste liquid container are connected to each other via a tube, and the new stirring part and the new waste liquid container are connected to each other via a tube. Then, the container attachment/detachment device 14 removes the closed container 50 from the tubes 72, 73 and 74 while maintaining the aseptic state inside the culture circuit 81 including the connection path 30 and removes the stirring part 32 from the tubes 73 and 74 and the tube 71 having one end connected to the closed container 60. Further, the container attachment/detachment device 14 connects the new closed container to the tubes 72, 73 and 74 while maintaining the aseptic state inside the new closed container including the new stirring part and the waste liquid container and connects the new stirring part to the tubes 73 and 74 and the tube 71 having one end connected to the closed container 60. As a result, the closed container 50, the stirring part 32 and the waste liquid container 35 are integrally removed from the culture circuit 81, and the new closed container, the stirring part and the waste liquid container are integrally connected to the culture circuit 81. Thereafter, the closed container 50 including the stirring part 32 and the waste liquid container 35, which was removed from the culture circuit 81, is unloaded from the first chamber 11 to the outside of the cell culture device 1, and the first opening/closing part 21 of the first chamber 11 is closed. As a result, the replacement of the closed container is completed (step S13). The loading and unloading of the closed container may be performed by an operator or may be performed automatically. When the loading and unloading of the closed container is performed automatically, for example, a robot arm or the like is separately arranged. Further, the volume of the new closed container loaded from the outside is larger than that of the closed container 60. This is the same as the above-described reason why the volume of the closed container 60 is larger than the volume of the closed container 50. Further, when the subculture is performed a plurality of times, the volume of the closed container newly loaded from the outside is larger than the volume of the closed container accommodated inside the first chamber 11 or the second chamber 12.

After the cell suspension is contained in the closed container 60, the process returns to step S3. That is, the valve V19 is opened by the controller 5. When an excess gas remains inside the closed container 60, the gas is discharged from the degassing part 61 attached to the closed container 60. Thereafter, the cell suspension contained in the closed container 60 is subjected to a culture step, a medium replacement step and a cell suspension recovery step in the second chamber 12 (steps S4 to S8).

When the medium replacement step (step S5) is performed on the closed container 60 accommodated in the second chamber 12, first, the controller 5 opens the valves V2 and V16 and drives the pump 101. As a result, the medium inside the closed container 60 is discharged to the waste liquid container 135 through the tube 76. At this time, the gas inside the waste liquid container 135 is released to the atmosphere through the degassing part 136. Thereafter, the controller 5 stops the pump 101 and closes the valves V2 and V16. Next, the controller 5 opens the valves V7 and V10 and drives the pump 102 to supply a fresh medium from the medium container 34 to the closed container 60 via the tube 74. Thereafter, the controller 5 stops the pump 102 and closes the valves V7 and V10.

Further, when the cell suspension recovery step (step S8) is performed on the closed container 60 accommodated in the second chamber 12, first, the controller 5 opens the valves V2 and V5 and drives the pump 101. As a result, the cell suspension inside the closed container 60 is moved to the stirring part 132 through the tube 72. Thereafter, the controller 5 stops the pump 101 and closes the valves V2 and V5. Subsequently, the controller 5 performs the supply of the medium to the stirring part 132 and/or the discharge of the excess cell suspension from the stirring part 132 so that the concentration of the cell suspension contained inside the stirring part 132 becomes equal to a preset concentration. When performing the supply of the medium, the controller 5 opens the valves V7 and V11 and drives the pump 102. As a result, a predetermined amount of medium is supplied from the medium container 34 to the stirring part 132 via the tube 74. When performing the discharge of the excess cell suspension, the controller 5 opens the valves V13 and V17 and drives the pump 101 to allow the CO₂ inside the gas tank 33 to flow into the stirring part 132. As a result, a part of the cell suspension inside the stirring part 132 is discharged to the waste liquid container 135 via the tube 76. At this time, the gas inside the waste liquid container 135 is released to the atmosphere through the degassing part 136. As a result, the concentration of the cell suspension contained in the stirring part 132 becomes equal to the preset concentration.

Step S13 and step S4 for culturing the cells inside the closed container 60 may be performed in parallel. Further, if the preset number of subcultures and the actual number of subcultures are the same, step S13 is not performed. That is, for example, when the preset number of subcultures is two, step S13 is not performed after the completion of the second subculture steps (S10 to S12).

In the case where subcultures is performed a plurality of times, the cell suspension is contained in the closed container arranged inside the second chamber 12 in the culture steps (steps S4 and S6) and the medium replacement step (step S5) after the odd-numbered subculture steps (steps S10 to S12). In the culture steps and the medium replacement step after the even-numbered subculture steps, the cell suspension is contained in the closed container arranged inside the first chamber 11. Further, in the step of replacing the closed container (step S13) after the odd-numbered subculture steps, the closed container arranged inside the first chamber 11 is replaced with a new closed container. In the step of replacing the closed container after the even-numbered subculture steps, the closed container arranged inside the second chamber 12 is replaced with a new closed container.

After the cell suspension recovery step (step S8) described above, if a preset number of subcultures has already been performed and no further subculture is performed (S9: NO), the cell suspension is harvested (Step S14), and the cell culture is completed.

The cell culture device 1 of the present embodiment includes: a chamber formed in a culture part 4; a connection path 30 configured to connect closed containers 50 and 60 accommodated inside the chamber to each other; a driving part 40 configured to move cells between the closed containers 50 and 60 via the connection path 30; and a container attachment/detachment device 14 configured to remove the closed container 50 as a cell movement source from tubes 72, 73 and 74 while maintaining an aseptic state inside a culture circuit 81 including the closed container 60 to which the cells are moved by the driving part 40 and the connection path 30, and connect a new closed container to the tubes 72, 73 and 74 of the culture circuit 81 while maintaining an aseptic state inside the new closed container. According to such a configuration, when the cells have been cultured in any of the closed containers, the driving part 40 can be driven to move the cells from the closed container 50 to the closed container 60 via the connection path 30, whereby the subculture can be performed. When the subculture is completed, the closed container 50 as a cell movement source can be unloaded from the chamber through an opening/closing part, and a new closed container can be loaded into the chamber through an opening/closing part. The new closed container can be connected to the tubes 72, 73 and 74 by the container attachment/detachment device 14 while maintaining an aseptic state. Therefore, even when the subculture is performed a plurality of times, it is not necessary to prepare closed containers to be used for the second and subsequent subcultures in the chamber in advance, which makes it possible to suppress an increase in the size of the cell culture device. Further, by repeating the replacement of the closed container, it is possible to cope with the increase in the number of subcultures, whereby the limit on the number of subcultures is substantially eliminated.

In the present embodiment, the chamber includes a first chamber 11 having a first opening/closing part 21 and configured to accommodate the closed container 50 therein and a second chamber having a second opening/closing part 22 and configured to accommodate the closed container 60 therein, and an environment adjustment part 13 configured to adjust internal environments of the first chamber 11 and the second chamber 12 is installed. In general, cells are affected by a culture environment. Therefore, in order to properly culture the cells, it is required to keep the internal environment of the cell culture device 1 constant. In the cell culture device 1, the opening/closing part of the chamber is opened at the time of replacing the closed container. Therefore, it is difficult to keep the internal environment of the chamber of the cell culture device 1 constant. According to the above-described configuration, for example, when subculture is performed from the closed container 50 accommodated in the first chamber 11 to the closed container 60 accommodated in the second chamber 12, the closed container 50 can be replaced by opening only the first opening/closing part 21 of the first chamber 11. At this time, if the second opening/closing part 22 of the second chamber 12 is kept in a closed state, the internal environment of the second chamber 12 in which the closed container 60 for performing a cell culture after the subculture is installed can be kept constant without being affected by the outside of the cell culture device 1.

In the present embodiment, the cell culture device 1 further includes: sensors 23 configured to detect temperatures inside the first chamber 11 and the second chamber 12, wherein the environment adjustment part 13 is configured to adjust the temperatures inside the first chamber 11 and the second chamber 12, and the environment adjustment part 13 adjusts the temperatures inside the first chamber 11 and the second chamber 12 to predetermined values when the cells are moved through the connection path 30. According to this configuration, the cells are moved between the closed containers installed in the respective chambers when the temperatures inside the first chamber 11 and the second chamber 12 are equal to the predetermined temperatures, whereby the cell culture temperature can be kept constant before and after subculture.

Although the preferred embodiments of the present disclosure have been described above, the present disclosure is not limited to these embodiments, and various modifications may be made within the recitations in the claims.

For example, in the above-described embodiment, the culture part 4 includes the first chamber 11 having the first opening/closing part 21 and the second chamber 12 having the second opening/closing part 22. Alternatively, one chamber having one opening/closing part may be arranged. However, in this case, when the opening/closing part is opened to replace the closed container, the temperature inside the chamber in which the closed containers 50 and 60 are arranged is affected by the outside of the cell culture device 1. Therefore, it is preferable that the cell culture device 1 includes the first chamber 11 in which the closed container 50 is arranged and the second chamber 12 in which the closed container 60 is arranged.

Further, when the culture part 4 includes two chambers, the respective chambers may be arranged at positions separated from each other, or one chamber may be partitioned into two chambers by a heat insulating plate or the like. Further, the number of chambers arranged in the culture part 4 may be three or more. However, it is preferable that the number of chambers is two in order to suppress the increase in the size of the device.

Further, the number of closed containers arranged inside the first chamber 11 and the second chamber 12 does not have to be one for each chamber, and a plurality of closed containers may be arranged in at least one of the two chambers. For example, one closed container may be arranged in the first chamber 11 and two closed containers may be arranged in the second chamber 12. In this case, in the subculture step, the cell suspension is moved from the closed container arranged in the first chamber 11 to the two closed containers in the second chamber 12 via the connection path 30.

Further, in the above-described embodiment, the volume of the closed container 60 is larger than the volume of the closed container 50. However, when a multi-layer container is used as the closed container, the number of layers of the closed container 60 may be larger than the number of layers of the closed container 50. Further, the volume of the closed container 60 does not necessarily have to be larger than the volume of the closed container 50. The volume of the closed container 50 and the volume of the closed container 60 may be the same, or the volume of the closed container 50 may be larger the volume of the closed container 60. However, for the reasons described above, the volume of the closed container 60 is generally larger than the volume of the closed container 50.

In the above-described embodiment, the environment adjustment part 13 can adjust the CO₂ concentration inside the first chamber 11 and the second chamber 12 based on the information detected by the sensors 23. However, when the closed containers 50 and 60 are made of a material that does not allow CO₂ to pass therethrough, the environment adjustment part 13 does not need to adjust the CO₂ concentrations inside the first chamber 11 and the second chamber 12. In this case, the environment adjustment part 13 adjusts only the temperatures inside the first chamber 11 and the second chamber 12 or adjusts the temperatures inside the first chamber 11 and the second chamber 12 and the predetermined internal environments excluding the CO₂ concentrations.

Further, the environment adjustment part 13 may be able to adjust the inside of the first chamber 11 and the inside of the second chamber 12 so as to have different internal environments from each other. This makes it possible, for example, to change the culture environment before and after subculture.

Further, in the above-described embodiment, the check valves are installed between the waste liquid container 35 and the degassing part 36 and between the waste liquid container 135 and the degassing part 136, respectively. Air filters may be installed instead the check valves. In this case, it is possible to prevent various germs and the like from entering the waste liquid container 35 or the waste liquid container 135 from the outside of the device, and it is possible to suppress the risk of various germs entering the culture circuit 81.

Further, air filters may be further installed between the closed container 50 and the degassing part 51 and between the closed container 60 and the degassing part 61. As a result, if the valve V18 or the valve V19 is opened when the gas inside the closed container 50 or 60 is discharged to the outside of the device, it is possible to suppress the risk of entry of various germs into the culture circuit 81 from the outside air.

Further, in the above-described embodiment, the stirring part 32, the closed container 50 and the closed container 60 are connected to the medium container 34 via the tube 73. Alternatively, the stirring part 32, the closed container 50 and the closed container 60 may be further connected to a container containing yet another reagent. For example, when a reagent is added to the closed container in the culture step or the subculture step, the stirring part 32, the closed container 50 and the closed container 60 may be connected to a reagent container containing the reagent. In this case, a valve is further installed between the reagent container and the pump 102.

In the above-described embodiment, the container attachment/detachment device 14 integrally removes the closed container 50, the stirring part 32 and the waste liquid container 35 from the culture circuit 81. However, the container attachment/detachment device 14 may remove only the closed container 50 from the culture circuit 81. In this case, the container attachment/detachment device 14 removes the closed container 50 from the tubes 71 to 75 and connects a new closed container loaded from the outside to the tubes 71 to 75. Further, the container attachment/detachment device 14 may integrally remove the closed container 50 and the stirring part 32 from the culture circuit 81, and the waste liquid container 35 may be kept connected to the culture circuit 81. In this case, the container attachment/detachment device 14 removes the closed container 50 from the tubes 72 to 75 and removes the stirring part 32 from the tubes 73 to 75. Then, the container attachment/detachment device 14 connects a new closed container loaded from the outside to the tubes 72 to 75 and connects a new stirring part to the tubes 73 to 75.

In the above-described embodiment, the opening and closing of the first opening/closing part 21 and the second opening/closing part 22 are controlled by the controller 5. However, the first opening/closing part 21 and the second opening/closing part 22 may be opened and closed by the operator.

Further, in order to prevent the medium from remaining inside the tube 74, a cleaning step may be performed after the medium replacement step. In this case, a gas communication part communicating with the outside air is installed between the valve V7 and the pump 102. Further, the gas communication part has a valve and communicates with the outside air when the valve is in an open state. For example, when the valves V7 and V8 are in an open state, after the fresh medium is sent from the medium container 34 to the closed container 50 via the tube 74, the cell culture device 1 performs a cleaning step to remove the medium remaining inside the tube 74. Specifically, the controller 5 stops the pump 102, closes the valve V7, and keeps the valve V8 in the open state. Then, the controller 5 opens the valve of the gas communication part installed between the valve V7 and the pump 102, and drives the pump 102 again. As a result, the gas flows from the gas communication part into the tube 74, and pushes the residual medium inside the tube 74 into the closed container 50. Thus, the residual medium inside the tube 74 can be removed. The above-mentioned cleaning step may be performed even after the fresh medium is supplied to the stirring part 32, the closed container 60 or the stirring part 132. In this case, the controller 5 opens the valve of the gas communication part installed between the valve V7 and the pump 102, opens the valve between the pump 102 and the container to which the fresh medium is sent, and drives the pump 102, thereby cleaning the inside of the tube 74. An air filter is installed at the tip of the valve of the gas communication part installed between the valve V7 and the pump 102 to suppress the risk of various germs entering the culture circuit 81 from the outside air.

Further, the degassing parts 51, 61, 36 and 136 may be capable of performing not only the discharge of the gas but also introduction of the outside air just like the gas communication part described above. In this case, opening/closing valves and air filters are arranged between the closed container 50 and the degassing part 51, between the closed container 60 and the degassing part 61, between the waste liquid container 35 and the degassing part 36, and between the waste liquid container 135 and the degassing part 136, respectively.

### EXPLANATION OF REFERENCE NUMERALS

1: cell culture device, 2: refrigerating storage part, 3: heating storage part, 4: culture part, 5: controller, 11: first chamber, 12: second chamber, 13: environment adjustment part, 14: container attachment/detachment device, 21: first opening/closing part, 22: second opening/closing part, 23: sensor, 30: connection path, 32, 132: stirring part, 33: gas tank, 35, 135: waste liquid container, 40; driving part, 50, 60: closed container, 71, 72, 73, 74, 75, 76: tube, 101, 102: pump

## Claims

1. A cell culture device for culturing cells in a closed container whose inside is kept in an aseptic state, comprising:
a chamber including an opening/closing part capable of being opened and closed;
a connection path configured to connect at least two closed containers accommodated in the chamber to each other and each having an inside kept in an aseptic state;
a driving part configured to move cells between the at least two closed containers via the connection path; and
a container attachment/detachment device configured to remove one of the at least two closed containers as a cell movement source from the connection path while maintaining an aseptic state inside another of the at least two closed containers to which the cells are moved by the driving part and inside the connection path, and connect a new closed container loaded into the chamber via the opening/closing part to the connection path instead of the removed closed container while maintaining an aseptic state inside the new closed container and inside the connection path.

2. The cell culture device of Claim 1, wherein the chamber includes a first chamber having a first opening/closing part and configured to accommodate at least one closed container therein and a second chamber having a second opening/closing part and configured to accommodate at least one closed container therein, and
wherein the cell culture device further comprises an environment adjustment part configured to adjust internal environments of the first chamber and the second chamber.

3. The cell culture device of Claim 2, further comprising:
a temperature sensor configured to detect temperatures inside the first chamber and the second chamber,
wherein the environment adjustment part is configured to adjust the temperatures inside the first chamber and the second chamber, and
the environment adjustment part adjusts the temperatures inside the first chamber and the second chamber to predetermined values when the cells are moved through the connection path.
